# EUROPEAN PATENT APPLICATION

(11) **EP 1 685 835 A2**
(43) Date of publication of application: **02.08.2006**
(21) Application number: 06007843.3
(22) Date of filing: 21.04.2000
(51) Int. Cl.: A61K 31/337, A61P 35/00

(54) **Long term administration of sub-therapeutic dose levels of pharmacologically active agents**

(30) Priority: 22.04.1999 US 130863 P
(62) Divisional of application: 00928296.3
(71) Applicant: American Bioscience, Inc., Santa Monica, CA 90403 (US)
(72) Inventor: Soon-Shiong, Patrick, Malibu, CA 90265 (US); Desai, Neil, P., Los Angeles, CA 90066 (US)
(74) Representative: Roques, Sarah Elizabeth

(57) **Abstract**

In accordance with the present invention, there are provided methods useful for the treatment of a subject having an infirmity. Invention methods comprise administering to a subject a sub-therapeutic dose level of a pharmacologically active agent (such as the anticancer drug paclitaxel) effective against an infirmity over an administration period sufficient to achieve a therapeutic benefit.

## Description

### FIELD OF THE INVENTION

The present invention relates to methods for the administration of pharmacologically active agents. In a particular aspect, the invention relates to methods for the *in vivo* delivery of pharmacologically active agents at sub-therapeutic dose levels.

### BACKGROUND OF THE INVENTION

Conventional chemotherapy in the treatment of cancer is usually performed by administering chemotherapeutic drugs via intravenous infusion or intravenous bolus injection. The objective of this type of intravenous administration is to achieve blood and tissue levels of the chemotherapeutic drug that are high enough so as to be cytotoxic to the tumor cells of the cancer being treated, and to maintain a therapeutically active level of agent.

However, there are problems associated with this type of intravenous administration. First, therapeutic (i.e., cytotoxic) levels of the chemotherapeutic drug commonly can be maintained only for a few days (i.e., less than 2-4 days) at best. Most chemotherapeutic drugs are cleared rapidly from the blood and tissues via normal *in vivo* clearance mechanisms. Second, these systemic modes of administration (i.e., intravenous administration) of high amounts of chemotherapeutic drugs commonly cause unnecessary toxicity reactions and adverse events in the subject being treated. Known intravenous administration modes commonly result in peak levels of the chemotherapeutic drug in the circulatory system which are far above the levels needed to kill the tumor cells of the cancer being treated.

These two problems are exemplified in the case of the anticancer agent paclitaxel (administered in its FDA approved formulation, Taxol^{™}). Taxol^{™} is administered to a human subject by continuous IV infusion over 1-24 hours. Unfortunately, blood levels of the drug following administration drop off rapidly and are undetectable after a few days following the treatment. In part to compensate for this drop off, administration of Taxol^{™} is then repeated after 3 weeks. In addition, levels of paclitaxel in the plasma of the subject over the first several hours of treatment may well exceed 5-10 µg/ml. This is substantially above the levels that are high enough to be cytotoxic to the tumor cells of the cancer being treated (i.e., 0.5-1.0 µg/ml). *In vitro* studies on tumor cell lines have shown paclitaxel to be active in the 0.5 µg/ml range.

Paclitaxel has emerged as one of the most active anticancer agents in clinical oncology. With paclitaxel, a 3-hour infusion is common practice.¹⁹⁻²² Clinical trials to date are aimed at optimizing the potential efficacy of this agent and includes studies investigating the effect of drug scheduling, that is, weekly therapy and duration of infusion, as well as combination trials with other chemotherapy agents and with radiation therapy. Often, these trials are designed to administer paclitaxel by 3-hour infusion; however, there is a growing body of information on the feasibility of 1-hour paclitaxel infusions, in both weekly and every 3- to 4-week chemotherapy regimens.⁽¹⁻⁸⁾ A number of studies have employed 1-hour infusion of paclitaxel. The Sarah Cannon Cancer Center, has documented experience with 1-hour paclitaxel infusions in more than 1100 patients.⁽²²⁾

Paclitaxel was first introduced into clinical trials in 1983 and was studied in a variety of infusion schedules, including 1-, 3-, 6-, and 24-hour infusion durations.⁹ Hypersensitivity reactions were observed in up to 18% of patients treated on the early phase I trials.¹⁰ In an effort to prevent hypersensitivity reactions, a prolonged infusion of paclitaxel was adopted based on fewer observed hypersensitivity reactions in patients receiving 6- or 24- hour infusions in phase I studies.¹⁰ The 24-hour administration schedule, with premedication, was selected for phase II trials. In 1992, paclitaxel gained Food and Drug Administration approval for the treatment of relapsed advanced ovarian cancer at a dose of 135 mg/m² administered over 24 hours.

Paclitaxel binds to the -subunit of tubulin, promoting and stabilizing the assembly of microtubules, which leads to abnormal tubulin polymerization and cell cycle arrest in the G₂-M phase.¹² Ultimately, cell death of paclitaxel-treated cells appears to occur via apoptosis.¹² Experiments conducted in vitro have demonstrated that very low concentrations of paclitaxel, on the order of 0.05 M, are effective in disrupting normal tubulin polymerization, and that cytotoxicity can be both concentration and schedule dependent. ^{12,13}

The pharmacokinetics of paclitaxel are nonlinear, with the peak plasma concentrations and the area under the curve (AUC) of the concentration versus time profile rising disproportionately with the dose.¹⁴⁻¹⁷ The effect is more pronounced with higher doses and shorter infusions of paclitaxel and appears to be directly related to saturable metabolism and elimination of the drug.¹⁷ Pharmacodynamic studies of paclitaxel indicate that neutropenia is not correlated with peak plasma concentrations above a threshold concentration (0.05 or 0.1 M).^{14,15,18} The duration of time at or above this threshold concentration is a function of both dose and schdule.

A large amount of clinical data has failed to substantiate preclinical predictions of the importance of schedule dependency in determining the clinical efficacy of paclitaxel. In breast and ovarian cancer, trials seeking to optimize the dose and the schedule of paclitaxel are ongoing.

Thus, there is a need for methods of administration of pharmacologically active agents (especially chemotherapeutic drugs) which can achieve therapeutic levels of the pharmacologically active agent over more than a few days (i.e., more than 2-4 days). In addition, there is a need for methods of administration of pharmacologically active agents (e.g., chemotherapeutic drugs) which do not cause unnecessary toxicity reactions and adverse events in a subject being treated due to the presence of substantially higher than therapeutic levels (e.g., levels that are cytotoxic to tumor cells in the subject being treated for a cancer) of the pharmacologically active agent.

### BRIEF DESCRIPTION OF THE INVENTION

In accordance with the present invention, methods have been developed for the treatment of a subject having an infirmity. Invention methods comprise administering to the subject sub-therapeutic dose levels (i.e., very low levels, such as levels below the conventionally accepted therapeutic dose) of a pharmacologically active agent effective against the infirmity. Surprisingly, it has been found that continuously administering pharmacologically active agents (especially chemotherapeutic agents) effective against infirmities at sub-therapeutic dose levels over long or extended periods is efficacious in the treatment of these infirmities. Invention methods of treatment can be applied systemically or locally, as required for the treatment of a variety of infirmities.

The present invention provides many advantages over the art. For example, the present invention provides methods of administration of pharmacologically active agents (especially chemotherapeutic drugs) which can achieve therapeutic levels of the pharmacologically active agent over more than a few days (i.e., more than 2-4 days). In addition, the present invention provides methods of administration of pharmacologically active agents (e.g., chemotherapeutic drugs) which do not cause unnecessary toxicity reactions and adverse events in a subject being treated due to the presence of substantially higher than therapeutic levels (i.e., levels that are cytotoxic to tumor cells in subject being treated for a cancer) of the pharmacologically active agent. Further, the present invention provides methods for treating a variety of infirmities via localized or systemic administration of sub-therapeutic dose levels of pharmacologically active agents (e.g., chemotherapeutic drugs) over extended administration times. Other advantages of the present invention can be readily recognized by those of ordinary skill in the art.

### DETAILED DESCRIPTION OF THE INVENTION

In accordance with the present invention, there are provided methods for the treatment of a subject having an infirmity. Invention methods comprise administering to the subject sub-therapeutic dose levels of a pharmacologically active agent effective against the infirmity.

Subjects contemplated for treatment in accordance with the present invention include humans, domesticated animals, animals useful for commercial or research purposes, and the like.

As utilized herein, the term "infirmity" includes diseases, injuries, conditions which adversely effect the health and/or well-being of a subject, and the like. Infirmities can be systemic or localized. Exemplary localized infirmities where the invention treatment can be applied include breast cancers, ovarian cancers, lung cancers, hepatic disease (primary or secondary), brain disease, bladder cancer, prostate cancer, any other type of cancer that is conventionally characterized as a local disease, and the like, and combinations of two or more thereof. In general, any localized infirmity that is accessible to the placement of a catheter, an implantable or portable infusion device, or a slow release delivery vehicle through which the pharmacologically active agent can be delivered is suitable for treatment in accordance with the present invention.

Administration of pharmacologically active agents contemplated for use in the practice of the present invention can be accomplished by a variety of routes, as are well known to those of skill in the art. Thus, exemplary routes of administration include topical, oral, intraarticular, intracisternal, intraocular, intraventricular, intrathecal, intravenous, intramuscular, intraperitoneal, intradermal/transdermal/subcutaneous, intratracheal/inhalational, rectal (i.e., via suppository), vaginal (i.e., via pessary), intracranial, intraurethral, intrahepatic, intraarterial, intratumoral, mucosal, and the like, as well as suitable combinations of two or more thereof.

Further, administration of pharmacologically active agents contemplated for use in the practice of the present invention can be systemic (i.e., administered to the subject as a whole via any of the above routes) or localized (i.e., administered to the specific location of the particular infirmity of the subject via any of the above routes).

Exemplary means for the systemic administration of pharmacologically active agents are well known to those of skill in the art, and include oral (i.e., with a sustained release formulation of the pharmacologically active agent), continuous IV infusion, infusion via bolus injection, infusion through in-dwelling catheters, and any other means which can function to deliver the pharmacologically active agent systemically to the subject suffering the infirmity, and the like, and suitable combinations of two or more thereof.

Exemplary means for the localized administration of pharmacologically active agents include catheters, implantable or portable infusion devices, slow release delivery vehicles, and any other means which can function to deliver the pharmacologically active agent to the localized area of the infirmity, and the like, and suitable combinations of two or more thereof.

Implantable or portable infusion devices contemplated for use in the practice of the present invention are well known to those of skill in the art, and include devices which can deliver precise and controlled amounts of the drug over extended periods. Typically, these are driven by electromagnetic force or osmotic force. Commonly, implantable infusion devices are capable of being periodically refilled, and of being able to receive the pharmacologically active agent in solid or liquid form.

Exemplary slow release delivery vehicles include, for example, pharmacologically active agent encapsulated in a colloidal dispersion system or in polymer stabilized crystals. Useful colloidal dispersion systems include nanocapsules, microspheres, beads, lipid-based systems, (including oil-in-water emulsions), micelles, mixed micelles, liposomes, and the like. The colloidal system presently preferred is a liposome or microsphere. Liposomes are artificial membrane vesicles which are useful as slow release delivery vehicles when injected or implanted. Some examples of lipid-polymer conjugates and liposomes are disclosed in U.S. Patent No. 5,631,018, which is incorporated herein by reference in its entirety. Other examples of slow release delivery vehicles are biodegradable hydrogel matrices (U.S. Patent No. 5,041,292), dendritic polymer conjugates (U.S. Patent No. 5,714,166), and multivesicular liposomes (Depofoam® , Depotech, San Diego, CA) (U. S. Patent Nos. 5,723,147 and 5,766,627). One type of microspheres suitable for encapsulating therapeutic agents for local injection (e.g., into subdermal tissue) is poly(D,L)lactide microspheres, as described by D. Fletcher, in *Anesth. Analg.* 84:90-94, 1997.

Besides delivering an effective therapeutic dose to the site of the infirmity and decreasing the chance of systemic toxicity, localized administration also decreases the exposure of the pharmacologically active agent to degradative processes, such as proteolytic degradation and immunological intervention via antigenic and immunogenic responses, as well as to systemic clearance processes, such as sequestration in the liver.

Sub-therapeutic dose levels contemplated for use in the practice of the present invention include actual levels of pharmacologically active agent (e.g., plasma levels for systemic administration, and infirm tissue levels for localized administration) that are lower than conventionally accepted plasma levels considered essential for successful treatment of the infirmity when the pharmacologically active agent is administered by conventional means (e.g., by continuous IV infusion or bolus injection).

As used herein, the term " therapeutic levels", is meant to be understood in a broad context in that the level of the pharmacologically active agent may not even be detectable in any measurable amount in any physiological body compartment (such as but not limited to blood, urine, sputum, or tissue levels), and yet demonstrate efficacy in preventing tumor progression, or even resulting in tumor regression.

In a multi-cycle treatment, sub-therapeutic dose levels can also include actual total doses of the pharmacologically active agent administered over a period of one cycle of the multi-cycle treatment that are lower than conventionally accepted total doses considered essential for successful treatment of the infirmity when the pharmacologically active agent is administered by conventional means (e.g., by continuous IV infusion or bolus injection).

For example, in the conventional treatment of cancer utilizing the drug paclitaxel (i.e., via the Taxol^{™} formulation), a dose of about 135-175 mg/m² is given every 3 weeks. The entire dose is usually given on the first day of the 3 week cycle. When administered in accordance with the present invention, paclitaxel can be continuously administered over the same 3 week period at a much lower total cumulative dose (e.g., 1-150 mg/m²). Furthemore an extremely low, continuos dose of a taxane (or any other pharmacologically active agent to treat the specific infirmity), can be administered for extremely prolonged periods (eg >lweek, >1month, 1 to 12 months, >lyear) at doses which are not detectable in any measurable, physiologial compartment of the human body. Specifically, for taxanes it may be possible to administer doses <1 mg/m2 for extremely prolonged periods with or without breaks in the cycle times.

The amount of the sub-therapeutic dose of pharmacologically active agents, either as an actual level of pharmacologically active agent in a subject or as a total dose of pharmacologically active agent administered over a period of one cycle of the multi-cycle treatment, is generally defined in relative terms (i.e., as a percentage amount (less than 100%) of the amount of pharmacologically active agent conventionally administered). This amount can vary over a wide range, expressed herein as a sub-therapeutic dose amount range. Typically, the low end point of this sub-therapeutic dose amount range is greater than or equal to about 1% of the amount of pharmacologically active agent conventionally administered. Another way to express acceptable values for the low end point of this sub-therapeutic dose amount range is as any integer percentage value, in the range from about 1% to less than about 98%, of the amount of pharmacologically active agent conventionally administered. Exemplary low end points of this sub-therapeutic dose amount range include 1%, 5%, 10%, 25%, 50%, 70%, 90%, 95%, and 98% of the amount of pharmacologically active agent conventionally administered. The corresponding upper end point of this sub-therapeutic dose amount range is generally less than or equal to about 99% of the amount of pharmacologically active agent conventionally administered. Another way to express acceptable values for this corresponding upper end point of the sub-therapeutic dose amount range is as any integer percentage value greater than the selected low end point of this range and in the range from greater than about 10% to about 99% of the amount of pharmacologically active agent conventionally administered. Exemplary high endpoints of this sub-therapeutic dose amount range include 10%, 20%, 30%, 50%, 75%, 90%, 95%, and 99% of the amount of pharmacologically active agent conventionally administered.

Sub-therapeutic dose levels can be administered over any period of time that is longer than the conventional time frame for administering the pharmacologically active agent. The time period for administration of sub-therapeutic dose levels of pharmacologically active agents can be defined in absolute terms (i.e., by a specific time period) or in relative terms (i.e., by a specific time increment in excess of the conventional time period for administration).

In absolute terms, the time period for administration of sub-therapeutic dose levels of pharmacologically active agents can vary over a wide range, expressed herein as a sub-therapeutic dose level administration time range. Typically, the low end point of this sub-therapeutic dose level administration time range is greater than or equal to about 2 days. Another way to express acceptable values for the low end point of this sub-therapeutic dose level administration time range is as any integer value of days in the range from about 2 days to less than about 365 days (i.e., 1 year). Exemplary low end points of this sub-therapeutic dose level administration time range include 2 days, 7 days, 14 days (i.e., 2 weeks), and 30 days (i.e., 1 month). The corresponding upper end point of this sub-therapeutic dose level administration time range is generally less than or equal to about 365 days (i.e., 1 year). Another way to express acceptable values for this corresponding upper end point of the sub-therapeutic dose level administration time range is as any integer day value greater than the selected low end point of this range and in the range from greater than about 7 days to about 365 days (i.e., 1 year). Exemplary high endpoints of this sub-therapeutic dose level administration time range include 90 days (i.e., 3 months), 180 days (i.e., 6 months), 270 days (i.e., 9 months), and 365 days (i.e., 1 year). In one embodiment, the sub-therapeutic dose level administration time is in a range from about 2 days to about 1 year, in a preferred range from about 7 days to about 9 months, or in a presently preferred range from about 2 weeks to about 3 months.

In relative terms, the time period for administration of sub-therapeutic dose levels of pharmacologically active agents can be defined as any time period greater than the time period conventionally employed for administering the pharmacologically active agent.

In the case of paclitaxel (administered via the conventional Taxol^{™} formulation), the conventional administration time is typically 1-24 hours of continuous intravenous infusion. Although paclitaxel has also been given on a 96 hour schedule, this 96 hour schedule is not universally practiced. Thus, the period for administration of a sub-therapeutic dose level of paclitaxel in accordance with the present invention refers to any period in excess of about 96 hours, i.e., in excess of 4 days.

Phannacologically active agents contemplated for use in the practice of the present invention include chemotherapeutic drugs, taxanes, epitholones, agents which modify microtubule activity or assembly, small molecule drugs, biologics (e.g., peptides and the like), proteins, antibodies, enzymes, antisense therapeutics, polynucleotides (e.g., DNA, RNA, and the like), synthetic polynucleotide constructs (e.g., for use in gene delivery), antiinfectives, antirejection drugs, and the like, and suitable combinations of any two or more thereof.

Examples of pharmacologically active agents contemplated for use in the practice of the present invention also include:
analgesics/antipyretics (e.g., aspirin, acetaminophen, ibuprofen, naproxen sodium, buprenorphine hydrochloride, propoxyphene hydrochloride, propoxyphene napsylate, meperidine hydrochloride, hydromorphone hydrochloride, morphine sulfate, oxycodone hydrochloride, codeine phosphate, dihydrocodeine bitartrate, pentazocine hydrochloride, hydrocodone bitartrate, levorphanol tartrate, diflunisal, trolamine salicylate, nalbuphine hydrochloride, mefenamic acid, butorphanol tartrate, choline salicylate, butalbital, phenyltoloxamine citrate, diphenhydramine citrate, methotrimeprazine, cinnamedrine hydrochloride, meprobamate, and the like);
anesthetics (e.g., cyclopropane, enflurane, halothane, isoflurane, methoxyflurane, nitrous oxide, propofol, and the like),
antiasthmatics (e.g., Azelastine, Ketotifen, Traxanox, and the like),
antibiotics (e.g., neomycin, streptomycin, chloramphenicol, cephalosporin, ampicillin, penicillin, tetracycline, and the like),
antidepressants (e.g., nefopam, oxypertine, doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluoxetine hydrochloride, doxepin hydrochloride, imipramine hydrochloride, imipramine pamoate, nortriptyline, amitriptyline hydrochloride, isocarboxazid, desipramine hydrochloride, trimipramine maleate, protriptyline hydrochloride, and the like);
antidiabetics (e.g., biguanides, hormones, sulfonylurea derivatives, and the like),
antifungal agents (e.g., griseofulvin, keloconazole, amphotericin B, Nystatin, candicidin, and the like),
antihypertensive agents (e.g., propanolol, propafenone, oxyprenolol, Nifedipine, reserpine, trimethaphan camsylate, phenoxybenzamine hydrochloride, pargyline hydrochloride, deserpidine, diazoxide, guanethidine monosulfate, minoxidil, rescinnamine, sodium nitroprusside, rauwolfia serpentina, alseroxylon, phentolamine mesylate, reserpine, and the like);
anti-inflammatories (e.g., (non-steroidal) indomethacin, naproxen, ibuprofen, ramifenaxone, piroxicam, (stemidal) cortisone, dexamethasone, fluazacort, hydrocortisone, prednisolone, prednisone, and the like),
antineoplastics (e.g., adriamycin, cyclophosphamide, actinomycin, bleomycin, duanorubicin, doxorubicin, epirubicin, mitomycin, methotrexate, fluorouracil, carboplatin, cannustine (BCNU), methyl-CCNU, cisplatin, etoposide, interferons, camptothecin and derivatives thereof, phenesterine, paclitaxel and derivatives thereof, taxotere and derivatives thereof, taxane and derivatives thereof, vinblastine, vincristine, tamoxifen, etoposide, piposulfan, and the like),
antianxiety agents (e.g., lorazepam, buspirone hydrochloride, prazepam, chlordiazepoxide hydrochloride, oxazepam, clorazepate dipotassium, diazepam, hydroxyzine pamoate, hydroxyzine hydrochloride, alprazolam, droperidol, halazepam, chlormezanone, dantrolene, and the like),
immunosuppressive agents (e.g., cyclosporine, azathioprine, mizoribine, FK506 (tacrolimus), and the like),
antimigraine agents (e.g., ergotamine tartrate, propanolol hydrochloride, isometheptene mucate, dichloralphenazone, and the like);
sedatives/hypnotics (e.g., barbiturates (e.g., pentobarbital, pentobarbital sodium, secobarbital sodium), benzodiazapines (e.g., flurazepam hydrochloride, triazolam, tomazeparm, midazolam hydrochloride, and the like);
antianginal agents (e.g., beta-adrenergic blockers, calcium channel blockers (e.g., nifedipine, diltiazem hydrochloride, and the like), nitrates (e.g., nitroglycerin, isosorbide dinitrate, pentaerythritol tetranitrate, erythrityl tetranitrate, and the like), and the like);
antipsychotic agents (e.g., haloperidol, loxapine succinate, loxapine hydrochloride, thioridazine, thioridazine hydrochloride, thiothixene, fluphenazine hydrochloride, fluphenazine decanoate, fluphenazine enanthate, trifluoperazine hydrochloride, chlorpromazine hydrochloride, perphenazine, lithium citrate, prochlorperazine, and the like);
antimanic agents (e.g., lithium carbonate and the like),
antiarrhythmics (e.g., bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, lidocaine hydrochloride, and the like);
antiarthritic agents (e.g., phenylbutazone, sulindac, penicillamine, salsalate, piroxicam, azathioprine, indomethacin, meclofenamate sodium, gold sodium thiomalate, ketoprofen, auranofin, aurothioglucose, tolmetin sodium, and the like);
antigout agents (e.g., colchicine, allopurinol, and the like);
anticoagulants (e.g., heparin, heparin sodium, warfarin sodium, and the like);
thrombolytic agents (e.g., urokinase, streptokinase, altoplase, and the like);
antifibrinolytic agents (e.g., aminocaproic acid and the like);
hemorheologic agents (e.g., pentoxifylline and the like);
antiplatelet agents (e.g., aspirin, empirin, ascriptin, and the like);
anticonvulsants (e.g., valproic acid, divalproate sodium, phenytoin, phenytoin sodium, clonazepam, primidone, phenobarbitol, phenobarbitol sodium, carbamazepine, amobarbital sodium, methsuximide, metharbital, mephobarbital, mephenytoin, phensuximide, paramethadione, ethotoin, phenacemide, secobarbitol sodium, clorazepate dipotassium, trimethadione, and the like);
antiparkinson agents (e.g., ethosuximide, and the like);
antihistamines/antipruritics (e.g., hydroxyzine hydrochloride, diphenhydramine hydrochloride, chlorpheniramine maleate, brompheniramine maleate, cyproheptadine hydrochloride, terfenadine, clemastine fumarate, triprolidine hydrochloride, carbinoxamine maleate, diphenylpyraline hydrochloride, phenindamine tartrate, azatadine maleate, tripelennamine hydrochloride, dexchlorpheniramine maleate, methdilazine hydrochloride, trimprazine tartrate, and the like);
agents useful for calcium regulation (e.g., calcitonin, parathyroid hormone, and the like);
antibacterial agents (e.g., amikacin sulfate, aztreonam, chloramphenicol, chloramphenicol palmitate, chloramphenicol sodium succinate, ciprofloxacin hydrochloride, clindamycin hydrochloride, clindamycin pahnitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, colistin sulfate, and the like);
antiviral agents (e.g., interferon gamma, zidowdine, amantadine hydrochloride, ribavirin, acyclovir, and the like);
antimicrobials (e.g., cephalosporins (e.g., cefazolin sodium, cephradine, cefaclor, cephapirin sodium, ceftizoxime sodium, cefoperazone sodium, cefotetan disodium, cefutoxime azotil, cefotaxime sodium, cefadroxil monohydrate, ceftazidime, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, cefuroxime sodium, and the like), penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G potassium, penicillin G procaine, methicillin sodium, nafcillin sodium, and the like), erythromycins (e.g., erythromycin ethylsuccinate, erythromycin, erythromycin estolate, erythromycin lactobionate, erythromycin siearate, erythromycin ethylsuccinate, and the like), tetracyclines (e.g., tetracycline hydrochloride, doxycycline hyclate, minocycline hydrochloride, and the like), and the like);
anti-infectives (e.g., GM-CSFand the like);
bronchodialators (e.g., sympathomimetics (e.g., epinephrine hydrochloride, metaproterenol sulfate, terbutaline sulfate, isoetharine, isoetharine mesylate, isoetharine hydrochloride, albuterol sulfate, albuterol, bitolterol, mesylate isoproterenol hydrochloride, terbutaline sulfate, epinephrine bitartrate, metaproterenol sulfate, epinephrine, epinephrine bitartrate), anticholinergic agents (e.g., ipratropium bromide), xanthines (e.g., aminophylline, dyphylline, metaproterenol sulfate, aminophylline), mast cell stabilizers (e.g., cromolyn sodium), inhalant corticosteroids (e.g., flurisolidebeclomethasone dipropionate, beclomethasone dipropionate monohydrate), salbutamol, beclomethasone dipropionate (BDP), ipratropium bromide, budesonide, ketotifen, salmeterol, xinafoate, terbutaline sulfate, triamcinolone, theophylline, nedocromil sodium, metaproterenol sulfate, albuterol, flunisolide, and the like);
hormones (e.g., androgens (e.g., danazol, testosterone cypionate, fluoxymesterone, ethyltostosterone, testosterone enanihate, methyltestosterone, fluoxymesterone, testosterone cypionate), estrogens (e.g., estradiol, estropipate, conjugated estrogens), progestins (e.g., methoxyprogesterone acetate, norethindrone acetate), corticosteroids (e.g., triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate methylprednisolone sodium succinate, hydrocortisone sodium succinate, methylprednisolone sodium succinate, triamcinolone hexacatonide, hydrocortisone, hydrocortisone cypionate, prednisolone, fluorocortisone acetate, paramethasone acetate, prednisolone tebulate, prednisolone acetate, prednisolone sodium phosphate, hydrocortisone sodium succinate, and the like), thyroid hormones (e.g., levothyroxine sodium and the like), and the like);
hypoglycemic agents (e.g., human insulin, purified beef insulin, purified pork insulin, glyburide, chlorpropamide, glipizide, tolbutamide, tolazamide, and the like);
hypolipidemic agents (e.g., clofibrate, dextrothyroxine sodium, probucol, lovastatin, niacin, and the like);
proteins (e.g., DNase, alginase, superoxide dismutase, lipase, and the like);
nucleic acids (e.g., sense or anti-sense nucleic acids encoding any therapeutically useful protein, including any of the proteins described herein, and the like);
agents useful for erythropoiesis stimulation (e.g., erythropoietin);
antiulcer/antireflux agents (e.g., famotidine, cimetidine, ranitidine hydrochloride, and the like);
antinauseants/antiemetics (e.g., meclizine hydrochloride, nabilone, prochlorperazine, dimenhydrinate, promethazine hydrochloride, thiethylperazine, scopolamine, and the like);
oil-soluble vitamins (e.g., vitamins A, D, E, K, and the like);
as well as other drugs such as mitotane, visadine, halonitrosoureas, anthrocyclines, ellipticine, and the like;
as well as suitable combinations of two or more thereof.

Pharmacologically active agents contemplated for administration in accordance with the present invention can further comprise one or more adjuvants which facilitate delivery, such as inert carriers, colloidal dispersion systems, and the like. Representative and non-limiting examples of such inert carriers include water, isopropyl alcohol, gaseous fluorocarbons, ethyl alcohol, polyvinyl pyrrolidone, propylene glycol, a gel-producing material, stearyl alcohol, stearic acid, spermaceti, sorbitan monooleate, methylcellulose, and the like, as well as suitable combinations of two or more thereof.

Pharmacologically active agents contemplated for administration in accordance with the present invention can also be formulated as a sterile injectable suspension according to known methods using suitable dispersing agents, wetting agents, suspending agents, or the like. The sterile injectable preparation may also be a sterile injectable solution or suspension in a non-toxic parenterally-acceptable diluent or solvent, for example, as a solution in 1,4-butanediol. Sterile, fixed oils are conventionally employed as a solvent or suspending medium. For this purpose any bland fixed oil may be employed, including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like, can be incorporated as required, or, alternatively, can comprise the formulation.

In accordance with the present invention, there are also provided methods for eliminating cancer cells in a subject having the cancer cells. Invention methods comprise administering to the subject a sub-therapeutic dose level of an antineoplastic agent over a suitable administration time. In one embodiment, the antineoplastic agent is paclitaxel.

In accordance with another embodiment of the invention, there are provided methods for administration of a pharmacologically active agent to a subject in need thereof so as to achieve therapeutic levels thereof for more than 4 days, said method comprising regularly administering said pharmacologically active agent at a sub-therapeutic dose level for greater than 4 days.

In accordance with yet another embodiment of the present invention, there are provided methods for administration of a pharmacologically active agent to a subject in need thereof without subjecting said subject to adverse events caused by higher than therapeutic levels of said pharmacologically active agent, said method comprising regularly administering said pharmacologically active agent at a sub-therapeutic dose level for a time sufficient to achieve a therapeutic effect.

In accordance with still another embodiment of the present invention, there are provided unit dosage formulations for the treatment of a subject having an infirmity, said unit dosage form comprising a sub-therapeutic dose level of a pharmacologically active agent effective against said infirmity.

All references cited herein are incorporated herein by reference.

The invention will now be described in greater detail by reference to the following non-limiting example.

### Example 1

### Long Term Administration of Paclitaxel

The mechanism of action of the drug paclitaxel has been well studied. It is known that paclitaxel binds and stabilizes microtubules in tumor cells thus preventing further replication. In rapidly dividing cells as in a tumor, different cells are in different phases of the cell cycle at any given time. The effect of paclitaxel treatment results in the accumulation of the cells in the G2/M phase of the cell cycle as a result of microtubule stabilization. Depending on the rate of division of these cells, it would take a finite period of exposure of these cells to paclitaxel to result in the entire dividing cell population to be synchronized and locked in the G2/M phase.

Accordingly, cells exposed to higher and higher levels of the drug over a short time do not necessarily respond more efficaciously to drug exposure. In fact it is likely that exposure to lower levels of the drug over a longer period may result in a better response and a better overall cell kill.

Thus, in the case of actual paclitaxel levels, if plasma levels of paclitaxel are maintained at 0.01-0.05 µg/ml (considered a sub-therapeutic dose level) over a period of a week or longer, significant benefit is obtained in the treatment of cancers responsive to paclitaxel.

In addition, in the case of total paclitaxel dose over one treatment cycle in the conventional multi-cycle treatment of cancer utilizing the drug paclitaxel (i.e., via the Taxol^{™} formulation), a dose of about 200-250 mg/m² is given every 3 weeks. The entire dose is usually given on the first day of the 3 week cycle. However, when paclitaxel is continuously administered over the same 3 week period at a much lower total cumulative dose (e.g., 50-150 mg/m²) in accordance with the present invention, significant benefit is obtained in the treatment of cancers responsive to paclitaxel.

While the invention has been described in detail with reference to certain preferred embodiments thereof, it will be understood that modifications and variations are within the spirit and scope of that which is described and claimed.

### REFERENCES PAGE

1. Hainsworth JD, Thompson DS, Greco FA. Paclitaxel by 1-hour infusion: an active drug in metastatic non-small-cell lung cancer. J Clin Oncol 1995;13:1609-1614.
2. Fennelly D, Aghajanian C, Shapiro F et al. Phase I and pharmacokinetic study of paclitaxel administered weekly in patients with relapsed ovarian cancer. J Clin Oncol 1997; 15: 187-192.
3. Klaassen U, Wilke H, Strumberg D et al. Phase I study with a weekly 1 h infusion of paclitaxel in heavily pretreated patients with metastatic breast and ovarian cancer. Eur J Cancer 1996;2A:547-549.
4. Seidman AD. Paclitaxel via weekly 1-h infusion: dose-density with enhanced therapeutic index. Semin Oncol 1998;12[suppl 1]:19-22.
5. Chang A, Hui L, Boros R et al. Phase I study of weekly one-hour paclitaxel treatment in advanced malignant disease [abstract]. Proc Am Soc Clin Oncol 1997;16:232a.
6. Hainsworth JD, Raefsky EL, Greco FA. Paclitaxel administered by a 1-hour infusion: a phase I-II trial comparing two schedules. Cancer J Sci Am 1995;1:281-287.
7. Hainsworth JD, Gray JR, Stroup SL et al. Paclitaxel, carboplatin and extended-schedule etoposide in the treatment of small-cell lung cancer; comparison of sequential phase II trials using different dose-intervals. J Clin Oncol 1997;15:3464-3470.
8. Hainsworth JD, Erland JB, Kalman LA et al. Carcinoma of unknown primary site treatment with 1-hour paclitaxel, carboplatin, and extended-schedule etoposide. J Clin Oncol 1997;15:2385-2393.
9. Arbuck SG, Christian MC, Fisherman JS et al. Clinical development of Taxol. J Natl Cancer Inst Monogr 1993;11-24.
10. Onetta N, Canetta R, Winograd B et al. Overview of Taxol safety. J Natl Cancer Inst Monogr 1993;131-139.
11. Eisenhauer EA, ten Bokkel Huinink WW, Swenerton KD. European-Canadian randomized trial of paclitaxel in relapsed ovarian cancer: high-dose versus low-dose and long versus short infusion. J Clin Oncol 1994;12:2654-2666.
12. Dorr RT. Pharmacology of the taxanes. Pharmacotherapy 1997;17:96S-104S.
13. Lopes NM, Adams EG, Pitts TW et al. Cell kill kinetics and cell cycle effects of taxol on human and hamster ovarian cell lines. Cancer Chemother Pharmacol 1993;32:235-242.
14. Gianni L, Kearns CM, Giani A et al. Nonlinear pharmacokinetics and metabolism of paclitaxel and its pharmacodynamic relationships in humans. J Clin Oncol 1995;13:180-190.
15. Huizing MT, Keung ACF, Rosing H et al. Pharmacokinetics of paclitaxel and metabolites in a randomized comparative study in platinum-pretreated ovarian cancer patients. J Clin Oncol 1993;11:2127-2135.
16. Sonnichsen DS, Hurwitz CA, Pratt CB et al. Saturable pharmacokinetics and paclitaxel pharmacodynamics in children. J Clin Oncol 1994;12:532-538.
17. Kearns CM, Gianni L, Egorin MJ. Paclitaxel pharmacokinetics and pharmacodynamic s. Semin Oncol 1995;22 [suppl 6]:16-23.
18. Ohtsu T, Sasaki Y, Tamura T et al. Clinical pharmacokinetics and pharmacodynamics of paclitaxel: a 3-hour infusion versus a 24-hour infusion. Clin Cancer Res 1995;1:599-606.
19. Langer C, Rosvold E, Millenson M et al. Paclitaxel by 1 or 24 hour infusion combined with carboplatin in advanced non-small cell lung carcinoma: a comparative analysis [abstract]. Proc Am Soc Clin Oncol 1997;16:452a.
20. Holmes FA, Valero V, Walters R et al. Phase III trial of paclitaxel administered over 3 or 96 hours for metastatic breast cancer [abstract]. Proc Am Soc Clin Oncol 1996;15:106a.
21. Holmes FA, Valero V, Buzdar AU et al. Final results: randomized phase III trial or paclitaxel by 3-hour versus 96-hour infusion in patients with metastatic breast cancer. The long and short of it [abstract]. Proc Am Soc Clin Oncol 1998;16:110a.
22. Greco FA, Thomas M, Hainsworth JD. One-Hour Paclitaxel Infusions: Review of Safety and Efficacy. Vol.5, No.3 May/June 1999, p.179-191

## Claims

1. Use of a pharmacologically active agent in the manufacture of a medicament for the treatment of a subject having an infirmity, wherein said agent is effective against said infirmity and is administered to said subject at a sub-therapeutic dose level.

2. Use according to claim 1, wherein said pharmacologically active agent is selected from the group consisting of chemotherapeutic drugs, taxanes, epitholones, agents which modify microtubule activity or assembly, small molecule drugs, biologics, peptides, antibodies, enzymes, antisense therapeutics polynucleotides, synthetic polynucleotide constructs, antiinfectives, antirejection drugs, analgesics/antipyretics, anesthetics, antiasthmatics, antibiotics, antidepressants, antidiabetics, antifungal agents, antihypertensive agents, anti-inflammatories, antineoplastics, antianxiety agents, immunosuppresive agents, antimigraine agents, sedatives/hypnotics, antianginal agents, antipsychotic agents, antimanic agents, antiarrhythmics, antiarthritic agents, antigout agents, anticoagulants, thrombolytic agents, antifibrinolytic agents, hemorheologic agents, antiplatelet agents, anticonvulsants, antiparkinson agents, antihistamines/antipruritics, agents useful for calcium regulation, antibacterial agents, antiviral agents, antimicrobials, anti-infectives, bronchodialators, hormones, hypoglycemic agents, hypolipidemic agents, proteins, nucleic acids, agents useful for erythropoiesis stimulation, antiulcer/antireflux agents, antinauseants/antiemetics, oil-soluble vitamins, mitotane, visadine, halonitrosoureas, anthrocyclines, and ellipticine.

3. Use according to claim 1, wherein said pharmacologically active agent is administered by one or more routes of administration selected from the group consisting of topical, oral, intraarticular, intracisternal, intraocular, intraventricular, intrathecal, intravenous, intramuscular, intraperitoneal, intradermal/transdermal/subcutaneous, intratracheal/inhalational, rectal, vaginal, intracranial, intraurethral, intrahepatic, intraarterial, intratumoral, and mucosal.

4. Use according to claim 1, wherein said pharmacologically active agent is administered systemically.

5. Use according to claim 1, wherein said pharmacologically active agent is administered locally.

6. Use according to claim 1, wherein said sub-therapeutic dose is administered over an administration time in the range from about 2 days to about 1 year.

7. Use according to claim 1, wherein said sub-therapeutic dose is administered over an administration time in the range from about 7 days to about 9 months.

8. Use according to claim 1, wherein said sub-therapeutic dose is administered over an administration time in the range from about 2 weeks to about 3 months.

9. Use according to claim 1 wherein said infirmity is breast cancer, ovarian cancer, lung cancer, hepatic disease, brain disease, bladder cancer or prostate cancer.

10. Use according to claim 1 wherein said subject is a human.

11. Use of an antineoplastic agent in the manufacture of a medicament for eliminating cancer cells in a subject having said cancer cells, wherein said agent is administered to said subject at a sub-therapeutic dose level.

12. Use according to claim 11 wherein said antineoplastic agent is paclitaxel.

13. Use of a pharmacologically active agent in the manufacture of a medicament for administration to a subject in need thereof so as to achieve therapeutic levels thereof for more than 4 days, wherein said medicament is regularly administered at a sub-therapeutic dose level of said pharmacologically active agent for greater than 4 days.

14. Use of a pharmacologically active agent in the manufacture of a medicament for administration to a subject in need thereof without subjecting said subject to adverse events caused by higher than therapeutic levels of said pharmacologically active agent, wherein said medicament is regularly administered at a sub-therapeutic dose level of said pharmacologically active agent for a time sufficient to achieve a therapeutic effect.

15. A unit dosage form for the treatment of a subject having an infirmity, said unit dosage form comprising a sub-therapeutic dose level of a pharmacologically active agent effective against said infirmity.

16. A unit dosage form according to claim 15, wherein the pharmacologically active agent in the unit dosage form is selected from the group consisting of chemotherapeutic drugs, taxanes, epitholones, agents which modify microtubule activity or assembly, small molecule drugs, biologics, peptides, antibodies, enzymes, antisense therapeutics, polynucleotides, synthetic polynucleotide constructs, antiinfectives, antirejection drugs, analgesics/antipyretics, anesthetics, antiasthmatics, antibiotics, antidepressants, antidiabetics, antifungal agents, antihypertensive agents, anti-inflammatories, antineoplastics, antianxiety agents, immunosuppresive agents, antimigraine agents, sedatives/hypnotics, antianginal agents, antipsychotic agents, antimanic agents, antiarrhythmics, antiarthritic agents, antigout agents, anticoagulants, thrombolytic agents, antifibrinolytic agents, hemorheologic agents, antiplatelet agents, anticonvulsants, antiparkinson agents, antihistamines/antipruritics, agents useful for calcium regulation, antibacterial agents, antiviral agents, antimicrobials, anti-infectives, bronchodialators, hormones, hypoglycemic agents, hypolipidemic agents, proteins, nucleic acids, agents useful for erthropoiesis stimulation, antiulcer/antireflux agents, antinauseants/antiemetics, oil-soluble vitamins, mitotane, visadine, halonitrosoureas, anthrocyclines, and ellipticine.

17. A unit dosage form according to claim 15, wherein the pharmacologically active agent is administered by one or more routes of administration selected from the group consisting of topical, oral, intraarticular, intracisternal, intraocular, intraventricular, intrathecal, intravenous, intramuscular, intraperitoneal, intradermal/transdermal/subcutaneous, intratracheal/inhalational, rectal (i.e., via suppository), vaginal (i.e., via pessary), intracranial, intraurethral, intrahepatic, intraarterial, intratumoral, and mucosal.

18. Use according to claim 12 wherein said sub-therapeutic dose is from 1% to 20% of the conventionally administered dose.

19. Use according to claim 12 wherein said paclitaxel is administered at less than the conventional dose of about 135-175 mg/m² every 3 weeks.

20. Use according to claim 12 which maintains a plasma level of paclitaxel of 0.01 - 0.05 µg/ml over a period of a week or longer.
